# EUROPEAN PATENT APPLICATION

(11) **EP 4 241 778 A1**
(43) Date of publication of application: **13.09.2023**
(21) Application number: 21872459.9
(22) Date of filing: 22.09.2021
(51) Int. Cl.: A61K 35/744, A23K 10/16, A23L 33/135, A61K 35/747, A61P 21/00, C12N 1/20

(54) **MUSCLE ATROPHY PREVENTION AGENT**

(30) Priority: 23.09.2020 JP 2020158699
(71) Applicant: Megmilk Snow Brand Co. Ltd., Sapporo-shi, Hokkaido 065-0043 (JP)
(72) Inventor: UKIBE, Ken, Sapporo-shi, Hokkaido 065-0043 (JP); KAWATA, Daisuke, Sapporo-shi, Hokkaido 065-0043 (JP); SETO, Yasuyuki, Sapporo-shi, Hokkaido 065-0043 (JP)
(74) Representative: Potter Clarkson
(86) International application number: PCT/JP2021/034680
(87) International publication number: WO 2022/065330

(57) **Abstract**

An object of the invention is to provide a novel technique for preventing muscle atrophy by directly promoting muscle synthesis or suppressing muscle degradation without through physical activity. An agent for preventing muscle atrophy containing a lactic acid bacterium having a muscle synthesis-promoting effect and/or a muscle degradation-suppressing effect, a treated product of the lactic acid bacterium or an extract thereof as an active ingredient in which the lactic acid bacterium having a muscle synthesis-promoting effect is *Lactobacillus gasseri* or the like and the lactic acid bacterium having a muscle degradation-suppressing effect is *Lactobacillus reuteri* or the like is provided.

## Description

### TECHNICAL FIELD

The present invention relates to an agent for preventing muscle atrophy containing a lactic acid bacterium having a muscle synthesis-promoting effect and/or a muscle degradation-suppressing effect, a treated product of the lactic acid bacterium or an extract thereof as an active ingredient. Moreover, the invention relates to a pharmaceutical product, a food or a drink or feed for preventing muscle atrophy containing the agent for preventing muscle atrophy.

### BACKGROUND ART

In Japan, where the population is aging extremely, the gap between the average lifespan and the healthy life years is a pressing issue. In particular, age-related decline in the functions of locomotor organs, namely so-called locomotive syndrome, is one of major causes for decreasing the QOL (quality of life) of the elderly and is a big issue also in view of the burden of care and the increase in medical expenses. Of locomotor organs, muscles in particular have an impact-absorbing action and a posture-stabilizing action in addition to the motor function of muscles themselves and are important locomotor organs also for preventing impairment of other locomotor organs. The muscle mass, however, starts to decrease at the age of around 30, and the percentage of humans having sarcopenia, which is a state with a decrease in the whole-body skeletal muscle mass, increases with age. It is reported that the percentage is around 25% among elderly people aged between 75 and 79 and exceeds 35% among people aged 80 or higher (Non-Patent Document 1). Accordingly, preventing muscle atrophy and maintaining the muscle mass in the elderly are essential factors to prolong the healthy life years and to improve the QOL of the elderly.

Even among young people, muscle atrophy progresses easily and adversely affects rehabilitation and the prognosis when the load to muscles decreases, for example, due to an injury- or disease-induced temporary bedridden state. Thus, preventing muscle atrophy and maintaining the muscle mass leads to improvement of the QOL of not only the elderly but also young people.

The muscle mass is regulated by the balance between muscle synthesis and muscle degradation. Therefore, to prevent muscle atrophy, a method of promoting muscle synthesis or suppressing muscle degradation may be used. In particular, when promotion of muscle synthesis and suppression of muscle degradation can be achieved at the same time, muscle atrophy can be prevented more effectively.

Here, as a method for preventing muscle atrophy and increasing the muscle mass, intake of protein serving as the raw material of muscles and branched-chain amino acids, which stimulate muscle synthesis signals, in addition to strength training is generally recommended. Moreover, there is the technique described in Patent Document 1. Patent Document 1 discloses that a physical activity-promoting agent containing *Lactobacillus gasseri* strain OLL2809 as an active ingredient promotes the physical activity and increases the muscle mass. In addition, Patent Document 2 discloses lactic acid bacterium strains of *Lactobacillus* which promote the growth of myoblasts and promote muscle repair.

Patent Document 3 discloses that *Lactobacillus curvatus* or *Lactobacillus amylovorus* has an action of suppressing muscle degradation caused by the expression of Atrogin-1.

Non-Patent Document 2 (Nutirients) discloses that bifidobacteria have an action of increasing muscles.

It is, however, not realistic for elderly people or bedridden patients to take strength training continuously. Moreover, protein functions mostly as a raw material in muscle synthesis, and even when the protein intake is increased, the effect is limited unless the muscle synthesis promotion pathway or the degradation suppression pathway in muscle cells is activated. In particular, it is believed that muscle synthesis response to branched-chain amino acids weakens in the elderly people, and it is required to separately activate the muscle synthesis promotion pathway or the degradation suppression pathway to prevent muscle atrophy.

Moreover, in Patent Document 1, the muscle mass is increased as a secondary effect of the promotion of physical activity, but the agent does not directly promote muscle synthesis or suppress muscle degradation. Patent Document 2 promotes muscle repair of damaged muscles but does not promote muscle synthesis or suppress muscle degradation. Although Patent Document 3 suppresses muscle degradation, only two limited lactic acid bacteria are disclosed. Non-Patent Document 2 merely discloses that specific bifidobacteria have a muscle-increasing action but does not describe lactic acid bacteria.

None of the documents discloses a lactic acid bacterium having both a muscle synthesis-promoting effect and a muscle degradation-suppressing effect.

### CITATION LIST

### PATENT LITERATURE

[Patent Document 1] JP2016-84358A
[Patent Document 2] WO2019/230957
[Patent Document 3] JP6339526B

### NON PATENT LITERATURE

[Non-Patent Document 1] JAMDA 2013, 14, 911-915
[Non-Patent Document 2] Nutrients 2020, 12, 219

### SUMMARY OF INVENTION

### TECHNICAL PROBLEM

An object of the invention is to provide a novel technique for preventing muscle atrophy by directly promoting muscle synthesis or suppressing muscle degradation without through physical activity or a novel technique for effectively preventing muscle atrophy by promoting muscle synthesis and suppressing muscle degradation at the same time.

### SOLUTION TO PROBLEM

Muscle synthesis is promoted through activation of the Akt-mTOR (mechanistic target of rapamycin) pathway in muscle cells. p70S6K protein is located downstream of the Akt-mTOR pathway and is activated through phosphorylation. Thus, the amount of phosphorylated p70S6K is used as an indicator of activation of muscle synthesis, and an increase in the amount of phosphorylated p70S6K means activation of muscle synthesis.

The ubiquitin-proteasome system is mainly responsible for muscle degradation, and Atrogin-1 and MuRF1 (muscle ring finger 1) are known as muscle-specific ubiquitin ligases. That is, muscle degradation is promoted when the gene expression levels of Atrogin-1 and MuRF1 increase, while muscle degradation is suppressed when the gene expression levels of Atrogin-1 and MuRF1 decrease.

As a result of intensive studies, the inventors have found that the amount of phosphorylated p70S6K in muscle cells is increased by adding *Streptococcus thermophilus, Lactococcus lactis* subsp. *cremoris, Lactobacillus rhamnosus* or *Lactococcus lactis* subsp. *lactis* to muscle cells and that the gene expression levels of Atrogin-1 and MuRF1 are decreased by adding *Lactobacillus mucosae, Lactobacillus fermentum* and *Pediococcus acidilactici.* The inventors also found that the amount of phosphorylated p70S6K in muscle cells increases and the gene expression levels of Atrogin-1 and MuRF1 decrease when *Lactobacillus delbrueckii* subsp. *lactis, Lactobacillus gasseri* or *Lactobacillus reuteri* is added, thereby completing the invention.

The invention relates to the invention of [1] to [10] below.
[1] An agent for preventing muscle atrophy comprising a lactic acid bacterium having a muscle synthesis-promoting effect and/or a muscle degradation-suppressing effect, a treated product of the lactic acid bacterium or an extract thereof as an active ingredient,
   wherein the lactic acid bacterium having a muscle synthesis-promoting effect is any one kind or more selected from the group consisting of *Lactobacillus gasseri, Streptococcus thermophilus, Lactobacillus rhamnosus, Lactococcus lactis, Lactobacillus delbrueckii* and *Lactobacillus reuteri,* and
   the lactic acid bacterium having a muscle degradation-suppressing effect is any one kind or more selected from the group consisting of *Lactobacillus reuteri, Lactobacillus mucosae, Lactobacillus fermentum*, *Pediococcus acidilactici, Lactobacillus delbrueckii* and *Lactobacillus gasseri.*
[2] The agent for preventing muscle atrophy described in [1], wherein the lactic acid bacterium having a muscle synthesis-promoting effect is *Lactococcus lactis* subsp. *cremoris* or *Lactococcus lactis* subsp. *lactis.*
[3] The agent for preventing muscle atrophy described in [1] or [2], wherein the lactic acid bacterium having a muscle synthesis-promoting effect is *Lactobacillus gasseri* strain SBT1848 (NITE BP-03075), *Streptococcus thermophilus* strain SBT1021A (FERM P-10658), *Lactococcus lactis* subsp. *cremoris* strain SBT1393 (NITE P-03278), *Lactobacillus rhamnosus* strain SBT2299 (NITE BP-02994), *Lactococcus lactis* subsp. *lactis* strain SBT2397 (NITE P-03080) or *Lactobacillus reuteri* strain SBT2970 (NITE BP-03282).
[4] The agent for preventing muscle atrophy described in [1], wherein the lactic acid bacterium having a muscle degradation-suppressing effect is *Lactobacillus reuteri* strain SBT2970 (NITE BP-03282), *Lactobacillus mucosae* strain SBT2958 (NITE P-02803), *Lactobacillus mucosae* strain SBT10038 (NITE P-03283), *Lactobacillus mucosae* strain SBT10043 (NITE BP-03187), *Lactobacillus fermentum* strain SBT1846 (NITE BP-03279), *Lactobacillus fermentum* strain SBT1859 (NITE P-02996), *Pediococcus acidilactici* strain SBT3331 (NITE BP-02991) or *Lactobacillus gasseri* strain SBT1848 (NITE BP-03075).
[5] An agent for preventing muscle atrophy comprising a lactic acid bacterium having a muscle synthesis-promoting effect and a muscle degradation-suppressing effect, a treated product of the lactic acid bacterium or an extract thereof as an active ingredient.
[6] The agent for preventing muscle atrophy described in [5], wherein the lactic acid bacterium having a muscle synthesis-promoting effect and a muscle degradation-suppressing effect is *Lactobacillus delbrueckii, Lactobacillus gasseri* or *Lactobacillus reuteri.*
[7] The agent for preventing muscle atrophy described in [6], wherein the lactic acid bacterium having a muscle synthesis-promoting effect and a muscle degradation-suppressing effect is *Lactobacillus delbrueckii* subsp. *lactis.*
[8] The agent for preventing muscle atrophy described in [6], wherein the lactic acid bacterium having a muscle synthesis-promoting effect and a muscle degradation-suppressing effect is *Lactobacillus delbrueckii* subsp. *lactis* strain SBT1371 (NITE BP-03277), *Lactobacillus delbrueckii* subsp. *lactis* strain SBT2002 (NITE BP-03280), *Lactobacillus delbrueckii* subsp. *lactis* strain SBT2080 (NITE BP-03281), *Lactobacillus gasseri* strain SBT1848 (NITE BP-03075) or *Lactobacillus reuteri* strain SBT2970 (NITE BP-03282).
[9] A pharmaceutical product, a food or a drink, a food with a function claim, a food for a specified health use, a nutritional supplement, a supplement or feed for preventing muscle atrophy comprising the agent for preventing muscle atrophy described in any one of [1] to [8].
[10] *Lactobacillus delbrueckii* subsp. *lactis* strain SBT1371 (NITE BP-03277), *Lactobacillus delbrueckii* subsp. *lactis* strain SBT2002 (NITE BP-03280), *Lactobacillus delbrueckii* subsp. *lactis* strain SBT2080 (NITE BP-03281), *Lactococcus lactis* subsp. *cremoris* strain SBT1393 (NITE P-03278), *Lactobacillus reuteri* strain SBT2970 (NITE BP-03282), *Lactobacillus mucosae* strain SBT10038 (NITE P-03283) and *Lactobacillus fermentum* strain SBT1846 (NITE P-03279) which are novel lactic acid bacteria.
   The invention of the present application further has the constitutions [11] to [18] below.
[11] A method for preventing muscle atrophy comprising a step of administering a lactic acid bacterium having a muscle synthesis-promoting effect and/or a muscle degradation-suppressing effect, a treated product of the lactic acid bacterium or an extract thereof to a subject,
   wherein the lactic acid bacterium having a muscle synthesis-promoting effect is any one kind or more selected from the group consisting of *Lactobacillus gasseri, Streptococcus thermophilus, Lactobacillus rhamnosus, Lactococcus lactis* and *Lactobacillus delbrueckii,* and
   the lactic acid bacterium having a muscle degradation-suppressing effect is any one kind or more selected from the group consisting of *Lactobacillus reuteri, Lactobacillus mucosae, Lactobacillus fermentum*, *Pediococcus acidilactici* and *Lactobacillus delbrueckii.*
[12] The method for preventing muscle atrophy described in [11], wherein the lactic acid bacterium having a muscle synthesis-promoting effect is *Lactococcus lactis* subsp. *cremoris* or *Lactococcus lactis* subsp. *lactis.*
[13] The method for preventing muscle atrophy described in [11] or [12], wherein the lactic acid bacterium having a muscle synthesis-promoting effect is *Lactobacillus gasseri* strain SBT1848 (NITE BP-03075), *Streptococcus thermophilus* strain SBT1021A (FERM P-10658), *Lactococcus lactis* subsp. *cremoris* strain SBT1393 (NITE P-03278), *Lactobacillus rhamnosus* strain SBT2299 (NITE BP-02994), *Lactococcus lactis* subsp. *lactis* strain SBT2397 (NITE P-03080) or *Lactobacillus reuteri* strain SBT2970 (NITE BP-03282).
[14] The method for preventing muscle atrophy described in [11], wherein the lactic acid bacterium having a muscle degradation-suppressing effect is *Lactobacillus reuteri* strain SBT2970 (NITE BP-03282), *Lactobacillus mucosae* strain SBT2958 (NITE P-02803), *Lactobacillus mucosae* strain SBT10038 (NITE P-03283), *Lactobacillus mucosae* strain SBT10043 (NITE BP-03187), *Lactobacillus fermentum* strain SBT1846 (NITE BP-03279), *Lactobacillus fermentum* strain SBT1859 (NITE P-02996), *Pediococcus acidilactici* strain SBT3331 (NITE BP-02991) or *Lactobacillus gasseri* strain SBT1848 (NITE BP-03075).
[15] A method for preventing muscle atrophy comprising a step of administering a lactic acid bacterium having a muscle synthesis-promoting effect and a muscle degradation-suppressing effect, a treated product of the lactic acid bacterium or an extract thereof to a subject.
[16] The method for preventing muscle atrophy described in [15], wherein the lactic acid bacterium having a muscle synthesis-promoting effect and a muscle degradation-suppressing effect is *Lactobacillus delbrueckii, Lactobacillus gasseri* or *Lactobacillus reuteri.*
[17] The method for preventing muscle atrophy described in [16], wherein the lactic acid bacterium having a muscle synthesis-promoting effect and a muscle degradation-suppressing effect is *Lactobacillus delbrueckii* subsp. *lactis.*
[18] The method for preventing muscle atrophy described in [16], wherein the lactic acid bacterium having a muscle synthesis-promoting effect and a muscle degradation-suppressing effect is *Lactobacillus delbrueckii* subsp. *lactis* strain SBT1371 (NITE BP-03277), *Lactobacillus delbrueckii* subsp. *lactis* strain SBT2002 (NITE BP-03280), *Lactobacillus delbrueckii* subsp. *lactis* strain SBT2080 (NITE BP-03281), *Lactobacillus gasseri* strain SBT1848 (NITE BP-03075) or *Lactobacillus reuteri* strain SBT2970 (NITE BP-03282).

### ADVANTAGEOUS EFFECTS OF INVENTION

Preventing muscle atrophy and maintaining the muscle mass lead to improvement of the QOL of not only the elderly but also young people. According to the invention, muscle atrophy can be prevented by promoting muscle synthesis or suppressing muscle degradation through intake of a specific lactic acid bacterium. Furthermore, through intake of a lactic acid bacterium which promotes muscle synthesis and suppresses muscle degradation at the same time, muscle atrophy can be prevented effectively.

### BRIEF DESCRIPTION OF DRAWINGS

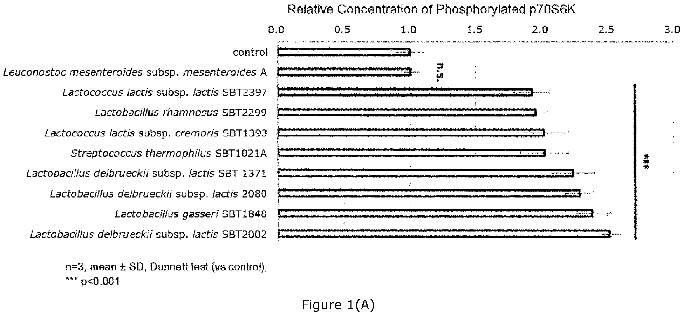

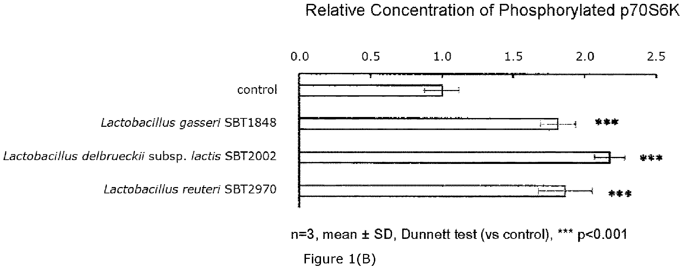

[Fig. 1] A graph showing the concentrations of phosphorylated p70S6K after adding lactic acid bacteria to mouse myoblast C2C12 cells according to Test Example 1, where the concentrations are relative concentrations to the average of the control group regarded as 1. In (A), the control group without the addition of the lactic acid bacterium homogenates, one lactic acid bacterium strain showing no muscle synthesis-promoting effect and eight lactic acid bacterium strains having a muscle synthesis-promoting effect were compared. In (B), the control group without the addition of the lactic acid bacterium homogenates and three lactic acid bacterium strains having a muscle synthesis-promoting effect were compared (SBT1848 and 2002 of the strains were tested also in (A)).

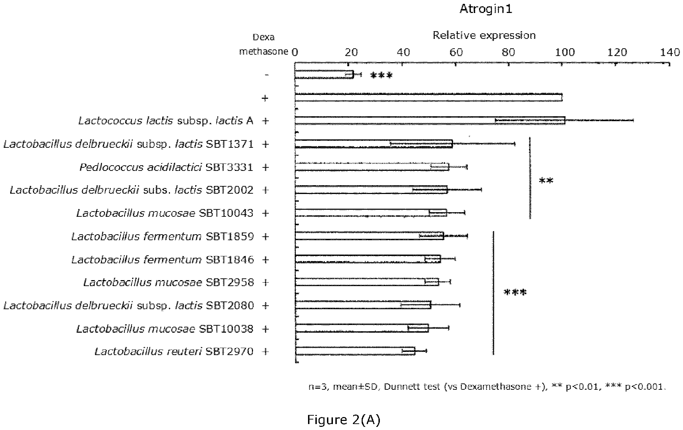

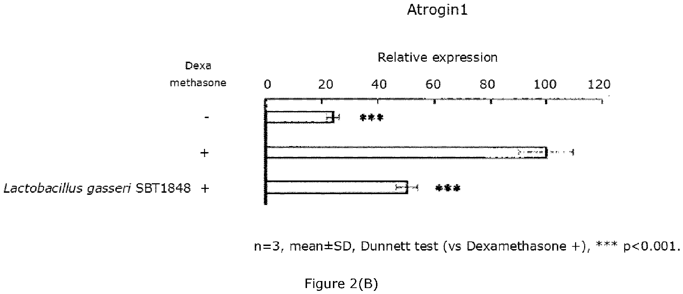

[Fig. 2] A graph comparing the muscle degradation-suppressing effects of lactic acid bacterium strains from the gene expression levels of Atrogin-1 after adding dexamethasone to C2C12 cells differentiated into myotube cells to induce muscle degradation according to Test Example 2. In (A), the group without the addition, the group to which only dexamethasone was added, the group to which dexamethasone and one lactic acid bacterium strain having no muscle degradation-suppressing effect were added and the groups to which dexamethasone and 10 lactic acid bacterium strains having a muscle degradation-suppressing effect were added were compared. In (B), the group without the addition, the group to which only dexamethasone was added and the group to which dexamethasone and one lactic acid bacterium strain having a muscle degradation-suppressing effect were added were compared.

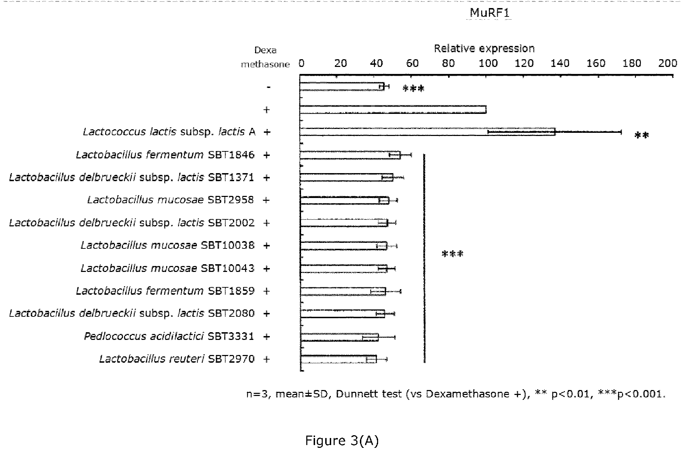

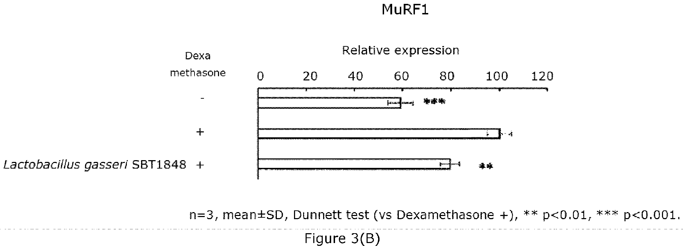

[Fig. 3] A graph comparing the muscle degradation-suppressing effects of lactic acid bacterium strains from the gene expression levels of MuRF-1 after adding dexamethasone to C2C12 cells differentiated into myotube cells to induce muscle degradation according to Test Example 3. In (A), the group without the addition, the group to which only dexamethasone was added, the group to which dexamethasone and one lactic acid bacterium strain having no muscle degradation-suppressing effect were added and the groups to which dexamethasone and 10 lactic acid bacterium strains having a muscle degradation-suppressing effect were added were compared. In (B), the group without the addition, the group to which only dexamethasone was added and the group to which dexamethasone and one lactic acid bacterium strain having a muscle degradation-suppressing effect were added were compared.

### DESCRIPTION OF EMBODIMENTS

The invention provides an agent for preventing muscle atrophy containing a lactic acid bacterium, a treated lactic acid bacterium product or an extract thereof (also simply referred to as a lactic acid bacterium or the like below) as an active ingredient.

The lactic acid bacterium or the like contained in the agent for preventing muscle atrophy of the invention is a specific lactic acid bacterium or the like having a muscle synthesis-promoting effect and is preferably obtained from any one kind or more selected from the group consisting of *Lactobacillus gasseri, Streptococcus thermophilus, Lactobacillus rhamnosus, Lactococcus lactis, Lactobacillus delbrueckii* and *Lactobacillus reuteri.*

Of these, *Lactococcus lactis* is further preferably *Lactococcus lactis* subsp. *cremoris* or *Lactococcus lactis* subsp. *lactis.*

Of the bacterial species, the lactic acid bacterium or the like is further preferably obtained from *Lactobacillus gasseri* strain SBT1848, *Streptococcus thermophilus* strain SBT1021A, *Lactococcus lactis* subsp. *cremoris* strain SBT1393, *Lactobacillus rhamnosus* strain SBT2299, *Lactococcus lactis* subsp. *lactis* strain SBT2397 or *Lactobacillus reuteri* strain SBT2970.

Moreover, the lactic acid bacterium or the like contained in the agent for preventing muscle atrophy of the invention is a specific lactic acid bacterium or the like having a muscle degradation-suppressing effect and is preferably obtained from any one kind or more selected from the group consisting of *Lactobacillus reuteri, Lactobacillus mucosae, Lactobacillus fermentum*, *Pediococcus acidilactici, Lactobacillus delbrueckii* and *Lactobacillus gasseri.*

Of the bacterial species, the lactic acid bacterium or the like is further preferably obtained from *Lactobacillus reuteri* strain SBT2970, *Lactobacillus mucosae* strain SBT2958, *Lactobacillus mucosae* strain SBT10038, *Lactobacillus mucosae* strain SBT10043, *Lactobacillus fermentum* strain SBT1846, *Lactobacillus fermentum* strain SBT1859, *Pediococcus acidilactici* strain SBT3331 or *Lactobacillus gasseri* strain SBT1848.

Furthermore, the lactic acid bacterium or the like contained in the agent for preventing muscle atrophy of the invention is a lactic acid bacterium or the like having a muscle synthesis-promoting effect and a muscle degradation-suppressing effect, and any lactic acid bacterium or the like having the effects can be used.

The lactic acid bacterium or the like having a muscle synthesis-promoting effect and a muscle degradation-suppressing effect is preferably obtained from *Lactobacillus delbrueckii, Lactobacillus reuteri* or *Lactobacillus gasseri. Lactobacillus delbrueckii* is further preferably obtained from *Lactobacillus delbrueckii* subsp. *lactis.*

Of the bacterial species, the lactic acid bacterium or the like is still further preferably obtained from *Lactobacillus delbrueckii* subsp. *lactis* strain SBT1371, *Lactobacillus delbrueckii* subsp. *lactis* strain SBT2002, *Lactobacillus delbrueckii* subsp. *lactis* strain SBT2080, *Lactobacillus reuteri* strain SBT2970 or *Lactobacillus gasseri* strain SBT1848.

The lactic acid bacterium or the like is preferably obtained from at least any of *Lactobacillus delbrueckii* subsp. *lactis, Lactobacillus gasseri, Streptococcus thermophilus, Lactococcus lactis* subsp. *cremoris, Lactobacillus rhamnosus, Lactococcus lactis* subsp. *lactis, Lactobacillus reuteri, Lactobacillus mucosae, Lactobacillus fermentum* and *Pediococcus acidilactici,* and one kind of the lactic acid bacteria or a mixture of more than one kind thereof can be used.

The lactic acid bacterium strains described above have been all deposited at NITE Patent Microorganisms Depositary (NPMD), National Institute of Technology and Evaluation. The information on deposit is described later in the specification.

The agent for preventing muscle atrophy in the invention has an action of preventing atrophy of muscles, and the presence or absence of an action of preventing muscle atrophy can be determined by the presence or absence of a muscle synthesis-promoting effect and/or a muscle degradation-suppressing effect.

The presence or absence of a muscle synthesis-promoting action can be determined, for example, by whether the subject lactic acid bacterium or the like increases the amount of phosphorylated p70S6K compared to that without the addition, as shown in the Examples described below.

The presence or absence of a muscle degradation-suppressing action can be determined, for example, by whether the subject lactic acid bacterium or the like decreases the gene expression levels of Atrogin-1 and MuRF1 compared to those without the addition, as shown in the Examples.

The medium for culturing the lactic acid bacterium according to the invention is not particularly limited as long as the lactic acid bacterium can be cultured in the medium, and any medium can be used.

The lactic acid bacterium according to the embodiment may be cultured in accordance with a general method for culturing a lactic acid bacterium, and a desired amount may be prepared. For example, lactic acid bacterium cells can be obtained by culturing the lactic acid bacterium using a synthetic medium such as MRS medium and centrifuging the obtained culture. The obtained bacterial cells may be used directly, or the bacterial cells may be used after subjecting to concentration, drying, lyophilization or fracturing. Dead bacterial cells obtained by heat drying the bacterial cells or the like can be used.

Because bacterial cells which have been subjected to concentration, drying, lyophilization or fracturing or dead bacterial cells obtained by heat drying or the like can be used as described above, the agent for preventing muscle atrophy of the embodiment can be widely used as a composition contained in a pharmaceutical product, a quasi-drug, a food or a drink, feed or the like.

For formulation, a generally used additive such as an excipient, a binder, a disintegrant and a flavoring agent may be appropriately mixed.

The food or the drink for preventing muscle atrophy of the invention may be a food or a drink containing the lactic acid bacterium or the like serving as an active ingredient and may be a food with a function claim, a food for a specified health use, a nutritional supplement, a supplement or the like. The food or the drink of the invention may be a milk beverage, yogurt, cheese, ice cream, a soft drink, butter, condensed milk, a cracker, infant formula, powdered formula, liquid formula, a condiment or the like. Moreover, the active ingredient of the invention may be added to an existing food or drink at the stage of raw materials, during the production or after the production. Furthermore, because the active ingredient of the invention is a lactic acid bacterium or the like, for example, the active ingredient itself of a fermented food obtained by fermentation with the lactic acid bacterium of the invention, such as yogurt, cheese and a milk beverage, is an example of the food or the drink for preventing muscle atrophy.

The amount of the agent for preventing muscle atrophy blended in such a food or a drink varies with the form, the dosage form, the symptom of the subject of administration, the body weight, the application or the like and thus is not particularly limited, but, for example, the agent can be blended at 0.001 to 100 (w/w)% if an example is to be given.

As the feed for preventing muscle atrophy, the active ingredient may be mixed in normal feed.

The subject of administration and the daily intake of the agent for preventing muscle atrophy according to the embodiment are not particularly limited, and the agent can be administered to a minor under the age of 20, an adult, an elderly person at the age of 65 or higher or the like, for example, when the subject of administration is a human. The daily intake varies with the age, the symptom, the body weight or the purpose and thus is not particularly limited, but, for example, the intake as the weight of the bacterial cells or the treated bacterial cells is 0.001 to 10 g, preferably 0.01 to 5 g if examples are to be given.

According to the embodiment, a novel agent for preventing muscle atrophy can be provided. The agent for preventing muscle atrophy contains a lactic acid bacterium or the like as an active ingredient, can be produced at a low price in a large volume and is highly safe. In particular, a lactic acid bacterium or the like which can promote muscle synthesis and suppress muscle degradation at the same time has a high effect of preventing muscle atrophy.

### EXAMPLES

As Examples of the invention, the results of evaluation of the muscle synthesis-promoting effect and the muscle degradation-suppressing effect using mouse myoblast C2C12 cells are briefly explained below. However, the invention is not limited to the embodiments of the Examples.

### [Preparation of Lactic Acid Bacterium Homogenates]

Glycerol stocks of bacterial strains were streaked on MRS medium plates and cultured at 37°C for three nights (64 hours). Next, one colony obtained from each medium plate was inoculated in 10 mL of MRS medium and cultured at 37°C or 30°C one night (16 hours). The bacterial cell cultures in a volume of 300 µL were subcultured in 10 mL of fresh MRS medium and cultured again at 37°C or 30°C one night (16 hours). Next, 3 mL of the bacterial cell cultures were inoculated in 100 mL of MRS medium and cultured at 37°C or 30°C one night (16 hours). The obtained bacterial cell cultures were centrifuged, and the precipitated bacterial cells were washed with PBS and ultrapure water. The bacterial cells were re-suspended in 4 mL of ultrapure water and frozen at -80°C. Through lyophilization using a lyophilizer (Tokyo Rikakikai Co., Ltd.), lyophilized bacterial cells were obtained. The obtained lyophilized bacterial cells were dissolved in PBS or DMEM medium (gibco) and fractured using a multi-beads shocker (Yasui Kikai Corporation), and thus lactic acid bacterium homogenates were prepared.

### [Test Example 1] Evaluation of p70S6K Phosphorylation-Enhancing Effect

Mouse myoblasts, C2C12 cells, were seeded on a 48-well culture plate (IWAKI) containing 10% FBS- and 1% penicillin-streptomycin-containing DMEM medium at 1.8×10⁴ cell/well. The cells were cultured under the conditions of 37°C and 5% CO₂ for two days to 80% to 90% confluency.

Next, the medium was replaced with FBS-free 1% penicillin-streptomycin-containing DMEM medium, and the cells were cultured for four hours. After the culture, the homogenates of the lactic acid bacteria shown in Fig. 1 which were suspended in 1% penicillin-streptomycin-containing DMEM medium were added thereto to 100 µg/mL. The same amount of 1% penicillin-streptomycin-containing DMEM medium which did not contain the lactic acid bacterium homogenates was added to the control group. The C2C12 cells were further cultured for two hours and then washed with PBS, and the cells were lysed using the Cell Extraction Buffer PTR included in p70S6K (pT389) SimpleStep ELISA (abcam). The cell solutions were recovered and centrifuged to obtain supernatants, and protein solution samples used for the subsequent ELISA were thus obtained.

ELISA was conducted using p70S6K (pT389) SimpleStep ELISA, which can detect phosphorylated p70S6K concentrations, and Pre-coated 384 well Microplate SimpleStep ELISA (abcam). The total protein concentrations of the cell solutions were determined by the BCA method, and the samples were diluted with the Cell Extraction Buffer PTR to a certain concentration. Together with a control sample for creating a calibration curve, 25 µL/well thereof were added to Pre-coated 384 well Microplate SimpleStep ELISA, and the colors were developed by the method described in the manual of p70S6K (pT389) SimpleStep ELISA. Using VARIOSKAN (Thermo Scientific), the absorbances at the major wavelength of 450 nm and a sub-wavelength of 620 nm were measured, and the differences in the absorbances between the wavelengths were determined. A calibration curve was created using the control sample, and the relative concentrations of phosphorylated p70S6K in the samples were calculated based on the differences.

The results of comparison between eight strains showing the activity and one strain showing no activity are shown in Fig. 1. Three strains of *Lactobacillus delbrueckii* subsp. *lactis,* namely strain SBT2002, strain SBT2080 and strain SBT1371, *Lactobacillus gasseri* strain SBT1848, *Streptococcus thermophilus* strain SBT1021A, *Lactococcus lactis* subsp. *cremoris* strain SBT1393, *Lactobacillus rhamnosus* strain SBT2299, *Lactococcus lactis* subsp. *lactis* strain SBT2397 and *Lactobacillus reuteri* SBT2970 increased the amount of phosphorylated p70S6K and showed a muscle synthesis-promoting action. On the other hand, *Leuconostoc mesenteroides* subsp. *mesenteroides* A did not increase the amount of phosphorylated p70S6K and showed no muscle synthesis-promoting action.

### [Test Example 2] Evaluation of Effect of Suppressing Atrogin-1 and MuRF1 Gene Expression

Mouse myoblasts, C2C12 cells, were seeded on a 48-well culture plate (IWAKI) containing 10% FBS- and 1% penicillin-streptomycin-containing DMEM medium at 1.8×10⁴ cell/well. The cells were cultured under the conditions of 37°C and 5% CO₂ for two days to 80% to 90% confluency.

Next, the medium was replaced with 2% horse serum-containing 1% penicillin-streptomycin-containing DMEM medium, and the cells were cultured for five days while changing the medium every two to three days to differentiate the C2C12 cells. Then, dexamethasone, which is known to increase the gene expression of Atrogin-1 and MuRF1, was added to the same medium at a concentration of 1 µM, and the lactic acid bacterium homogenates shown in Fig. 2 and Fig. 3 were added at 100 µg/mL. After culturing for 24 hours, the medium was removed, and 200 µL/well of Sepasol-RNA I Super G (Nacalai Tesque, Inc.) was added to the cells. The cells were fractured by pipetting and recovered. RNA was extracted from the recovered liquids, and the cDNA was synthesized from about 500 ng of the RNA as the template using ReverTra Ace qPCR RT Master Mix with gDNA remover (TOYOBO). Then, using THUNDERBIRD SYBR qPCR Mix (TOYOBO) and the primers for amplifying the genes, the gene expression levels ofAtrogin-1 and MuRF1 were determined with ViiA7 Real-time PCR system (Thermo Fisher Scientific). As the internal control, Gapdh gene was used. The sequences of the primers used are shown in Table 1.

**[Table 1]**

| Primer Sequences of Each Genes | | | | |
|---|---|---|---|---|
| | *Atrogin1* | F | GGCGGACGGCTGGAA | SEQ ID NO:1 |
| | | R | CAGATTCTCCTTACTGTATACCTCCTTGT | SEQ ID NO:2 |
| | *MuRF1* | F | ACGAGAAGAAGAGCGAGCTG | SEQ ID NO:3 |
| | | R | CTTGGCACTTGAGAGGAAGG | SEQ ID NO:4 |
| | *Gapdh* | F | ACCCAGAAGACTGTGGATGG | SEQ ID NO:5 |
| | | R | TTCAGCTCTGGGATGACCTT | SEQ ID NO:6 |

The results of comparison between the strains showing the activity and the strain showing no activity are shown in Figs. 2 and 3. Three strains of *Lactobacillus delbrueckii* subsp. *lactis,* namely strain SBT2002, strain SBT2080 and strain SBT1371, *Lactobacillus reuteri* strain SBT2970, *Lactobacillus mucosae* strain SBT2958, *Lactobacillus mucosae* strain SBT10038, *Lactobacillus mucosae* strain SBT10043, *Lactobacillus fermentum* strain SBT1846, *Lactobacillus fermentum* strain SBT1859, *Pediococcus acidilactici* strain SBT3331 and *Lactobacillus gasseri* strain SBT1848 decreased the gene expression levels of both Atrogin-1 and MuRF1 and showed a muscle degradation-suppressing action. On the other hand, *Lactobacillus lactis* subsp. *lactis* A did not decrease the gene expression levels of Atrogin-1 and MuRF1 and showed no muscle degradation-suppressing action.

From the above results, it was found that the lactic acid bacteria described in the Test Examples have a muscle synthesis-promoting action or a muscle degradation-suppressing action. In particular, *Lactobacillus delbrueckii* subsp. *lactis, Lactobacillus reuteri* and *Lactobacillus gasseri* subjected to the tests all showed both activities of a muscle synthesis-promoting action and a muscle degradation-suppressing action.

The invention is not limited to the bacterial strains described in the Examples and is highly likely to be widely applied to the same bacterial species.

### INDUSTRIAL APPLICABILITY

According to the invention, muscle atrophy can be prevented by promoting muscle synthesis or suppressing muscle degradation through intake of a specific lactic acid bacterium, and muscle atrophy can be prevented effectively through intake of a lactic acid bacterium which promotes muscle synthesis and suppresses muscle degradation at the same time. The agent for preventing muscle atrophy of the invention can improve the QOL of not only the elderly but also young people.

### ACCESSION NUMBER

### [Reference to Deposited Biological Material]

(1) Strain SBT1371
   (i) Name and Address of Depositary to Which the Biological Material was Deposited
      NITE Patent Microorganisms Depositary, National Institute of Technology and Evaluation (2-5-8 Kazusakamatari, Kisarazu-shi, Chiba (postal code 292-0818))
   (ii) Date of Deposit of Biological Material to Depositary of (i)
      September 15, 2020 (transferred to international deposit on September 10, 2021)
   (iii) Accession Number Given to Deposit by Depositary of (i)
      NITE BP-03277
(2) Strain SBT2002
   (i) Name and Address of Depositary to Which the Biological Material was Deposited Same as in (1) above.
   (ii) Date of Deposit of Biological Material to Depositary of (i)
      September 15, 2020 (transferred to international deposit on September 10, 2021)
   (iii) Accession Number Given to Deposit by Depositary of (i)
      NITE BP-03280
(3) Strain SBT2080
   (i) Name and Address of Depositary to Which the Biological Material was Deposited
      Same as in (1) above.
   (ii) Date of Deposit of Biological Material to Depositary of (i)
      September 15, 2020 (transferred to international deposit on September 10, 2021)
   (iii) Accession Number Given to Deposit by Depositary of (i)
      NITE BP-03281
(4) Strain SBT1393
   (i) Name and Address of Depositary to Which the Biological Material was Deposited
      Same as in (1) above.
   (ii) Date of Deposit of Biological Material to Depositary of (i)
      September 15, 2020
   (iii) Accession Number Given to Deposit by Depositary of (i)
      NITE P-03278
(5) Strain SBT2970
   (i) Name and Address of Depositary to Which the Biological Material was Deposited
      Same as in (1) above.
   (ii) Date of Deposit of Biological Material to Depositary of (i)
      September 15, 2020 (transferred to international deposit on September 10, 2021)
   (iii) Accession Number Given to Deposit by Depositary of (i)
      NITE BP-03282
(6) Strain SBT10038
   (i) Name and Address of Depositary to Which the Biological Material was Deposited
      Same as in (1) above.
   (ii) Date of Deposit of Biological Material to Depositary of (i)
      September 15, 2020
   (iii) Accession Number Given to Deposit by Depositary of (i)
      NITE P-03283
(7) Strain SBT1846
   (i) Name and Address of Depositary to Which the Biological Material was Deposited
      Same as in (1) above.
   (ii) Date of Deposit of Biological Material to Depositary of (i)
      September 15, 2020
   (iii) Accession Number Given to Deposit by Depositary of (i)
      NITE P-03279
(8) Strain SBT10043
   (i) Name and Address of Depositary to Which the Biological Material was Deposited Same as in (1) above.
   (ii) Date of Deposit of Biological Material to Depositary of (i)
      March 27, 2020 (transferred to international deposit on March 23, 2021)
   (iii) Accession Number Given to Deposit by Depositary of (i)
      NITE BP-03187
(9) Strain SBT1848
   (i) Name and Address of Depositary to Which the Biological Material was Deposited Same as in (1) above.
   (ii) Date of Deposit of Biological Material to Depositary of (i)
      November 25, 2019 (transferred to international deposit on September 10, 2021)
   (iii) Accession Number Given to Deposit by Depositary of (i)
      NITE BP-03075
(10) Strain SBT1021A
   (i) Name and Address of Depositary to Which the Biological Material was Deposited
      International Patent Organism Depositary, National Institute of Technology and Evaluation (2-5-8 Kazusakamatari, Kisarazu-shi, Chiba (postal code 292-0818))
   (ii) Date of Deposit of Biological Material to Depositary of (i)
      April 13, 1989
   (iii) Accession Number Given to Deposit by Depositary of (i)
      FERM P-10658
(11) Strain SBT2299
   (i) Name and Address of Depositary to Which the Biological Material was Deposited
      Same as in (1) above.
   (ii) Date of Deposit of Biological Material to Depositary of (i)
      June 26, 2019 (transferred to international deposit on July 13, 2020)
   (iii) Accession Number Given to Deposit by Depositary of (i)
      NITE BP-02994
(12) Strain SBT2397
   (i) Name and Address of Depositary to Which the Biological Material was Deposited
      Same as in (1) above.
   (ii) Date of Deposit of Biological Material to Depositary of (i)
      November 25, 2019
   (iii) Accession Number Given to Deposit by Depositary of (i)
      NITE P-03080
(13) Strain SBT2958
   (i) Name and Address of Depositary to Which the Biological Material was Deposited
      Same as in (1) above.
   (ii) Date of Deposit of Biological Material to Depositary of (i)
      October 31, 2018
   (iii) Accession Number Given to Deposit by Depositary of (i)
      NITE P-02803
(14) Strain SBT1859
   (i) Name and Address of Depositary to Which the Biological Material was Deposited
      Same as in (1) above.
   (ii) Date of Deposit of Biological Material to Depositary of (i)
      June 26, 2019
   (iii) Accession Number Given to Deposit by Depositary of (i)
      NITE P-02996
(15) Strain SBT3331
   (i) Name and Address of Depositary to Which the Biological Material was Deposited
      Same as in (1) above.
   (ii) Date of Deposit of Biological Material to Depositary of (i)
      June 26, 2019 (transferred to international deposit on July 13, 2020)
   (iii) Accession Number Given to Deposit by Depositary of (i)
      NITE BP-02991

## Claims

1. An agent for preventing muscle atrophy comprising a lactic acid bacterium having a muscle synthesis-promoting effect and/or a muscle degradation-suppressing effect, a treated product of the lactic acid bacterium or an extract thereof as an active ingredient,
wherein the lactic acid bacterium having a muscle synthesis-promoting effect is any one kind or more selected from the group consisting of *Lactobacillus gasseri, Streptococcus thermophilus, Lactobacillus rhamnosus, Lactococcus lactis, Lactobacillus delbrueckii* and *Lactobacillus reuteri,* and
the lactic acid bacterium having a muscle degradation-suppressing effect is any one kind or more selected from the group consisting of *Lactobacillus reuteri, Lactobacillus mucosae, Lactobacillus fermentum*, *Pediococcus acidilactici, Lactobacillus delbrueckii* and *Lactobacillus gasseri.*

2. The agent for preventing muscle atrophy according to claim 1, wherein the lactic acid bacterium having a muscle synthesis-promoting effect is *Lactococcus lactis* subsp. *cremoris* or *Lactococcus lactis* subsp. *lactis.*

3. The agent for preventing muscle atrophy according to claim 1 or 2, wherein the lactic acid bacterium having a muscle synthesis-promoting effect is *Lactobacillus gasseri* strain SBT1848 (NITE BP-03075), *Streptococcus thermophilus* strain SBT1021A (FERM P-10658), *Lactococcus lactis* subsp. *cremoris* strain SBT1393 (NITE P-03278), *Lactobacillus rhamnosus* strain SBT2299 (NITE BP-02994), *Lactococcus lactis* subsp. *lactis* strain SBT2397 (NITE P-03080) or *Lactobacillus reuteri* strain SBT2970 (NITE BP-03282).

4. The agent for preventing muscle atrophy according to claim 1, wherein the lactic acid bacterium having a muscle degradation-suppressing effect is *Lactobacillus reuteri* strain SBT2970 (NITE BP-03282), *Lactobacillus mucosae* strain SBT2958 (NITE P-02803), *Lactobacillus mucosae* strain SBT10038 (NITE P-03283), *Lactobacillus mucosae* strain SBT10043 (NITE BP-03187), *Lactobacillus fermentum* strain SBT1846 (NITE P-03279), *Lactobacillus fermentum* strain SBT1859 (NITE P-02996), *Pediococcus acidilactici* strain SBT3331 (NITE BP-02991) or *Lactobacillus gasseri* strain SBT1848 (NITE BP-03075).

5. An agent for preventing muscle atrophy comprising a lactic acid bacterium having a muscle synthesis-promoting effect and a muscle degradation-suppressing effect, a treated product of the lactic acid bacterium or an extract thereof as an active ingredient.

6. The agent for preventing muscle atrophy according to claim 5, wherein the lactic acid bacterium having a muscle synthesis-promoting effect and a muscle degradation-suppressing effect is *Lactobacillus delbrueckii, Lactobacillus gasseri* or *Lactobacillus reuteri.*

7. The agent for preventing muscle atrophy according to claim 6, wherein the lactic acid bacterium having a muscle synthesis-promoting effect and a muscle degradation-suppressing effect is *Lactobacillus delbrueckii* subsp. *lactis.*

8. The agent for preventing muscle atrophy according to claim 6, wherein the lactic acid bacterium having a muscle synthesis-promoting effect and a muscle degradation-suppressing effect is *Lactobacillus delbrueckii* subsp. *lactis* strain SBT1371 (NITE BP-03277), *Lactobacillus delbrueckii* subsp. *lactis* strain SBT2002 (NITE BP-03280), *Lactobacillus delbrueckii* subsp. *lactis* strain SBT2080 (NITE BP-03281), *Lactobacillus gasseri* strain SBT1848 (NITE BP-03075) or *Lactobacillus reuteri* strain SBT2970 (NITE BP-03282).

9. A pharmaceutical product, a food or a drink, a food with a function claim, a food for a specified health use, a nutritional supplement, a supplement or feed for preventing muscle atrophy comprising the agent for preventing muscle atrophy according to any one of claims 1 to 8.

10. *Lactobacillus delbrueckii* subsp. *lactis* strain SBT1371 (NITE BP-03277), *Lactobacillus delbrueckii* subsp. *lactis* strain SBT2002 (NITE BP-03280), *Lactobacillus delbrueckii* subsp. *lactis* strain SBT2080 (NITE BP-03281), *Lactococcus lactis* subsp. *cremoris* strain SBT1393 (NITE P-03278), *Lactobacillus reuteri* strain SBT2970 (NITE BP-03282), *Lactobacillus mucosae* strain SBT10038 (NITE P-03283) and *Lactobacillus fermentum* strain SBT1846 (NITE P-03279) which are novel lactic acid bacteria.
